# EUROPEAN PATENT APPLICATION

(11) **EP 4 553 066 A1**
(43) Date of publication of application: **14.05.2025**
(21) Application number: 23383151.0
(22) Date of filing: 10.11.2023
(51) Int. Cl.: C07D 233/88

(54) **IMPROVED PROCESS FOR THE SYNTHESIS OF IMEPITOIN**

(71) Applicant: Justesa Imagen S.A.U., 28823 Coslada, Madrid (ES)
(72) Inventor: ESTEVEZ LORA, Raul, 28823 Coslada, Madrid (ES); GARCIA RAMOS, Alvaro, 28823 Coslada, Madrid (ES); MARTINEZ GRAU, Maria Angeles, 28823 Coslada, Madrid (ES)
(74) Representative: Elzaburu S.L.P.

(57) **Abstract**

The present invention describes an improved process for the preparation of imepitoin by means of an efficient preparation of its intermediate 1-(4-chlorophenyl)imidazolidine-2,4-dione followed by condensation with morpholine in the presence of ammonium chloride. Particularly, the invention relates to a process which provides imepitoin in high yield by removing the water generated in the reaction using a continuous process to distil, dry and recirculate the dried morpholine back to the reactor. More particularly, the invention relates to a process that provides imepitoin with very high purity by a simple washing with toluene and water. Still more particularly, the invention relates to a cost-effective process that is scalable for industrial application.

## Description

### FIELD OF THE INVENTION

The present invention describes an improved process for the preparation of imepitoin by means of an efficient preparation of its intermediate 1-(4-chlorophenyl)imidazolidine-2,4-dione followed by condensation with morpholine in the presence of ammonium chloride. Therefore, the present invention belongs to the field of the synthesis processes of pharmaceutical compounds.

### BACKGROUND OF THE INVENTION

Imepitoin is chemically known as 1-(4-chlorophenyl)-1 ,5-dihydro-4-(4-morpholinyl)-2*H*-imidazol-2-one. It is marketed by Boehringer Ingelheim under the brand name Pexion^{®}.

Imepitoin is a centrally acting substance used in veterinary medicine to treat epilepsy and noise aversion in dogs. It acts as a low-affinity partial agonist at the benzodiazepine binding site of the GABA_{A} receptor. It is the first compound with such mechanism approved for the treatment of canine epilepsy, and for the reduction of anxiety and fear associated with noise phobia in dogs. In addition, imepitoin has a weak calcium channel blocking effect which may contribute to its anticonvulsant properties.

The known synthetic strategies for the preparation of imepitoin (B) describe the condensation of the intermediate 1-(4-chlorophenyl)imidazolidine-2,4-dione (A) with morpholine.

Imepitoin was first disclosed in patent DE19532668. This patent describes the small-scale synthesis of imepitoin by refluxing 1-(4-chlorophenyl)imidazolidine-2,4-dione (A) and morpholine (23 equivalents) in the presence of *p*-toluenesulfonic acid as catalyst. A conversion lower than 60% was obtained by removing the water formed during the reaction in a Soxhlet extractor filled with a water binding solid (e.g. calcined sodium sulfate, magnesium sulfate, NaOH, KOH, zeolites).

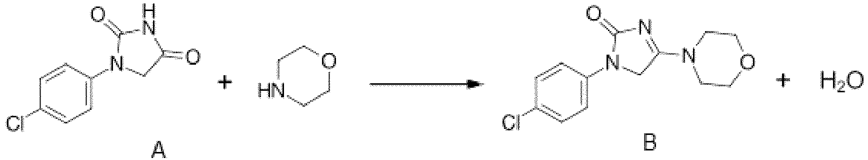

The product was isolated by distillation to half volume, cooling in an ice bath and filtration. The solid contained approximately 40% of the starting material that was removed by extraction with hot acetone. In addition, the product required crystallization from n-propanol. This method provides a lower yield than desirable for an industrial process and presents a lot of difficulties for industrial use: the use of a Soxhlet equipment is not available at manufacturing scale, the extraction with hot acetone to remove the unreacted starting material is not an industrially common operation, and the crystallization in n-propanol requires large solvent volumes due to the poor solubility of imepitoin in n-propanol.

Patent DE19532668 does not describe the preparation of the intermediate 1-(4-chlorophenyl)imidazolidine-2,4-dione (A).

J. Med. Chem. 2006, volume 49, pages 1855-1866, describes the preparation of imepitoin (B) by refluxing 1-(4-chlorophenyl)imidazolidine-2,4-dione (A) and morpholine (20 equivalents) in the presence of morpholine hydrochloride (2 equivalents). After cooling the mixture to room temperature, a solid precipitated. The solid was isolated by filtration, washed with water and crystallized from alcohols. This method is described at lab scale and provides 67% yield, quite moderate for a potential industrial process. In addition, morpholine hydrochloride is not a cheap chemical, and the crystallization in alcohols requires large solvent volumes due to the poor solubility of imepitoin in alcohols.

J. Med. Chem. 2006, volume 49, pages 1855-1866, does not describe the preparation of the intermediate 1-(4-chlorophenyl)imidazolidine-2,4-dione (A).

Patent WO2022153263 describes the preparation of imepitoin (B) by a similar procedure refluxing 1-(4-chlorophenyl)imidazolidine-2,4-dione (A) and morpholine (6 equivalents) in the presence of morpholine hydrochloride (2 equivalents). After cooling the mixture to 35 °C, methanol is added to precipitate a solid which is filtered and washed sequentially with methanol, water and methanol. The product is crystallized in DMSO and methanol to obtain imepitoin (B) with 53 % yield, quite moderate for a potential industrial process. In addition, morpholine hydrochloride is not a cheap chemical, and the crystallization in DMSO/methanol requires large solvent volumes of a non-optimal mixture for industrial use.

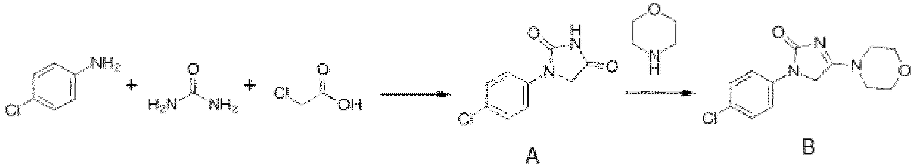

Patent WO2022153263 describes the preparation of the intermediate 1-(4-chlorophenyl)imidazolidine-2,4-dione (A) in one step by suspending 4-chloroaniline in xylene, adding urea and chloroacetic acid, and heating the system to 80 °C for 2 h, and then to 125 °C for 2 h. After a tedious work-up procedure, 1-(4-chlorophenyl)imidazolidine-2,4-dione (A) is obtained in 21% yield being this process quite inefficient for industrial use.

Khimiya Geterotsiklicheskikh Soedinenii, 1978, volume 1, pages 87-89, and Russian Journal of Organic Chemistry 2011, volume 47, pages 960-963 use this one step process from 4-chloroaniline, urea and chloroacetic acid in the absence of solvent to prepare 1-(4-chlorophenyl)imidazolidine-2,4-dione (A) with 53% and 62% yield respectively. In this method, the process of heating the solids to 120-130 °C in the lack of solvent does not lend itself to industrial use.

J. Prakt. Chem. 1926, volume 113, pages 233-267, describes the preparation of 1-phenylhydantoin in three steps by heating aniline, chloroacetic acid and sodium acetate in water. The intermediate aminophenylacetic acid was obtained in 32% yield only, due to the formation of phenylaminodiacetic acid. Once purified, aminophenylacetic acid was transformed into 1-phenylhydantoin by reaction with potassium cyanate, cyclization with hydrochloric acid and crystallization from alcohol. The overall yield was 19%.

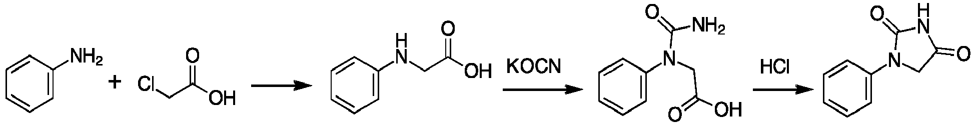

Patent EP0116825 used a similar procedure for the preparation of 1-phenylhydantoin. The treatment of an aqueous solution of commercially available N-phenylglycine with potassium cyanate and catalytic acetic acid at 60 °C followed by addition of hydrochloric acid, heating to 90 °C, filtration and crystallization provided 1-phenylhydantoin in 25% yield.

European Journal of Medicinal Chemistry 2009, volume 44, pages 3471-3479 used an analog experimental procedure in two steps starting from ethyl *N*-phenylglycinate for the preparation of 1-phenylhydantoin in 40% yield.

Drug Research 1968, volume 1, pages 189-196, describes the synthesis of 1-(4-chlorophenyl)-imidazolidine-2,4-dione (A) in 70% yield by refluxing 4-chlorophenylurea, ethyl chloroacetate and sodium ethoxide in ethanol. This reaction has two major disadvantages, the formation of by-products and the need to prepare 4-chlorophenylurea due to the high price and low availability at industrial level.

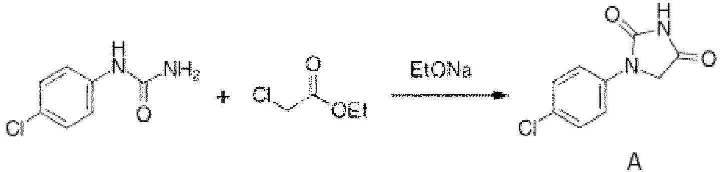

The Journal of Pharmaceutical Sciences 1973, volume 62, pages 340-341, describes the acid-catalyzed reflux of 4-chlorophenylurea and glyoxal in ethanol followed by fractional recrystallization to obtain 1-(4-chlorophenyl)-imidazolidine-2,4-dione (A) in 30% yield, being this process quite inefficient for industrial use.

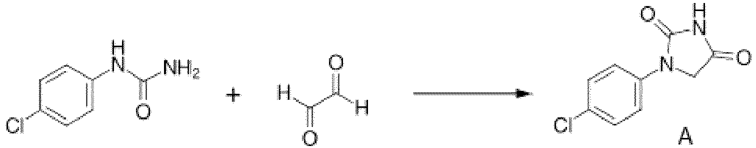

Tetrahedron Letters 2011, volume 52, pages 6148-6151, describes the synthesis of 1-(4-chlorophenyl)-imidazolidine-2,4-dione (A) by heating dibutyl phosphate with methyl *N*-cyano-2-(4-chloroanilino)acetate at 100 °C without solvent. Although this type of cyclization provides 85% yield, the method presents a lot of difficulties for industrial use: the solvent-free conditions, the cost of dibutyl phosphate, and the use of the *N*-cyano intermediate which is prepared by reaction of methyl 2-(4-chloroanilino)acetate with cyanogen bromide, a very toxic chemical that may cause cyanide poisoning.

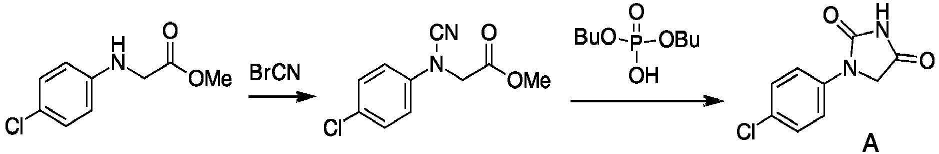

### DESCRIPTION OF THE INVENTION

The present invention solves the drawbacks of the known procedures for obtaining imepitoin, describing a cost effective and scalable process which prepares the title compound and the corresponding intermediates in high yield and purity without using any chromatographic purification or crystallization process. The procedure described below presents the advantages of using large-scale and cost-effective reaction conditions that minimize and remove impurities with simple isolation techniques, not requiring crystallization and allowing to obtain imepitoin with very high purity and overall yield. Therefore, the present invention describes an improved methodology to obtain imepitoin.

In a first aspect, the present invention provides a process for preparing high purity imepitoin which comprises the following steps:
a) obtention of a compound of formula (I) by an alkylation reaction 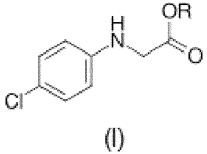 wherein R is selected from H, (C1-C4)-alkyl (preferably an ethyl group), allyl or benzyl, said alkylation reaction comprising the following steps:
   a1) reaction of 4-chloroaniline with a base selected from potassium carbonate, sodium carbonate, potassium acetate or sodium acetate and an additive selected from potassium or sodium iodide, in a solvent selected from acetonitrile, tetrahydrofuran, dimethylformamide, acetone, or an alcohol, at room temperature, for 15-60 min.,
   a2) addition of a compound of formula (II) 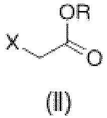 wherein X is a halogen or a leaving group, R is as previously defined, and heating at a reaction temperature between 50-80 °C, for 3-24 h,
   a3) addition of water, cooling at room temperature to precipitate compound (I),
   a4) distillation of the mixture ethanol/water and addition of water,
   a5) isolation of compound (I) by filtration and washing with a solvent,
b) obtention of a compound of formula (III) 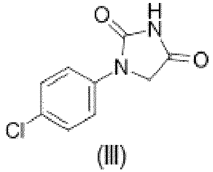 by an Urech-type hydantoin synthesis which comprises the following steps:
   b1) reaction of compound of formula (I) with potassium or sodium cyanate, in a solvent selected from acetic acid or hydrochloric acid, at a reaction temperature between 100-120 °C, for 4-24 h.,
   b2) cooling at room temperature, to precipitate compound (III),
   b3) isolation of compound (III) by filtration, washing with a solvent, and drying,
c) obtention of imepitoin by an acid-catalyzed reversible reaction which comprises the following steps:
   c1) reaction of morpholine as reactant and solvent, with ammonium chloride, at a reaction temperature between 110-140 °C, for 0.5-1.5 h,
   c2) addition of the compound (III) to the mixture of morpholine and ammonium chloride at a temperature between 110-140 °C,
   c3) heating the mixture to a temperature between 110-140 °C,
   c4) distillation of morpholine, drying with a desiccant agent, and return of the dried morpholine to the reaction mixture that it is maintained at a temperature between 110-140 °C, for 6-12 h,
   c5) cooling the reaction mixture to a temperature between 100-120 °C,
   c6) slow addition of toluene and morpholine to the reaction mixture at a temperature between 100-120 °C,
   c7) cooling the reaction mixture at room temperature to precipitate imepitoin,
   c8) filtration of the solid and resuspension in a solvent such as toluene, ethyl acetate, acetone, acetonitrile, THF, ethanol and iso-propanol, and most preferably toluene, with stirring at 20-55 °C, preferably room temperature, for 1-2 h, preferably 1 h,
   c9) filtration of the solid, washing with a solvent, preferably water, and drying under vacuum at a temperature between 40-60 °C, preferably 50 °C.

In a preferred embodiment, in step a1) the base is potassium carbonate, the additive is potassium iodide, the solvent is ethanol, at room temperature, for 30 min.

In a preferred embodiment, in step a2) X is chlorine, R is ethyl, the T is 60 ºC and the time is 5h.

In a preferred embodiment, the cooling in step a3) and/or in step b2) and/or in step c8) is until room temperature.

In a preferred embodiment, in step a5) the washing is carried out with water.

In a preferred embodiment, in step b1) the compound of formula (I) is reacted with potassium cyanate, the solvent is acetic acid, at a T of 110 ºC, for 6h.

In a preferred embodiment, in step b3) the washing is carried out with water and the drying is carried out under vacuum at the temperature of 50 °C.

In a preferred embodiment, the step c1) is carried out at a T of 120 ºC for 1h.

In a preferred embodiment, the step c2) is carried out at a T of 120 ºC.

In a preferred embodiment, the heating in step c3) is carried out at a T of 130 ºC.

In a preferred embodiment, in step c4) the drying is carried out through a column filled with activated 3Å molecular sieves and the reaction mixture is maintained at a T of 130 ºC for 8h.

As used herein, the term "alkyl" means a saturated, monovalent unbranched or branched hydrocarbon chain. Examples of alkyl groups include, but are not limited to, (C1-C4)-alkyl groups, such as methyl, ethyl, propyl, isopropyl, n-butyl, s-butyl, isobutyl, t-butyl. Ethyl group is preferred.

As used herein, the term "allyl" means a 2-propenyl group.

As used herein, the term "benzyl" means a -CH₂-aryl group where aryl is a substituted or unsubstituted phenyl ring.

As used herein, the term "room temperature" may be considered a temperature between 20 and 30 ºC.

The synthetic route of a specific embodiment of the process of the invention is provided in the following scheme:

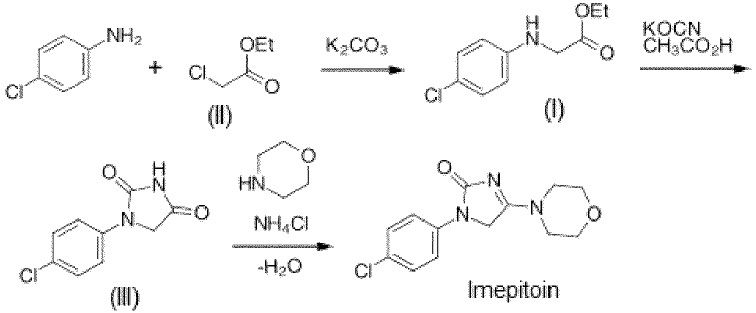

Advantages of the invention:
- The process is high yielding (68% overall yield)
- The process produces the title product with very high purity (>99.5% by HPLC, actually 99.8%) with no crystallization needed
- The process is cost effective
- The process is simple and scalable for industrial application

Known processes which do not include the step of drying the morpholine are not so advantageous as the process of the invention, as longer reaction time was required, yield was much lower, and purification process had to be repeated three times to obtain imepitoin with the same purity. A comparison is shown in one of the examples. These results highlight the importance of drying the morpholine continuously during the synthesis to obtain imepitoin as a solid with very high yield and purity after a simple purification process.

### EXAMPLES

### Example 1

### Synthesis of ethyl 2-(4-chloroanilino)acetate

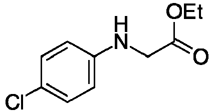

4-Chloroaniline (100 g, 0.78 mol) was dissolved in ethanol (200 mL) into a mechanically stirred reactor. Potassium carbonate (109.42 g, 0.79 mol) and potassium iodide (130.13 g, 0.78 mol) were added, and the mixture was stirred at room temperature for 30 min. Ethyl chloroacetate (84.74 mL, 0.78 mol) was added in one portion and the reaction was heated to 60 °C for 5 h. Water (600 mL) was added, and the mixture was allowed to cool to room temperature. As the temperature decreased, a cream-colored solid started to precipitate. When room temperature was reached, 400 mL of the mixture ethanol/water were distilled. Then, 300 mL of water were added, and again 300 mL of the solvent mixture was distilled. Finally, the solid was isolated by filtration and washed with water (500 mL), obtaining the desired compound as a cream-colored solid (175.75 g with 10% of water determined by KF, 94% yield on anhydrous base). The wet solid was used in the next step without drying. MS (ES+) *m*/*z*: 214 (M+H)⁺.

### Example 2

### Synthesis of 1-(4-chlorophenyl)imidazolidine-2,4-dione

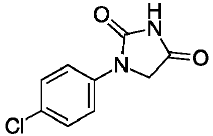

Wet ethyl 2-(4-chloroanilino)acetate (158.17 g on anhydrous base, 0.74 mol) was loaded into a mechanically stirred reactor. Acetic acid (1.05 L, 9.90 mol, 80% v/v) was loaded under stirring. Potassium cyanate (120.10 g, 1.48 mol) was slowly added to the solution in portions. The mixture was then heated to reflux and maintained at that temperature for 6 h. The reaction mixture was cooled to room temperature and a white solid precipitated. The solid was then isolated by filtration and washed with water (160 mL). The wet solid was transferred to an oven and dried under vacuum at 50 °C. The desired compound was obtained as a white solid (123.83 g, 79% yield). MS (ES+) *m*/*z*: 211 (M+H)⁺. The purity of the product was greater than 99%. Purity was determined by HPLC (Luna C18 column, 250 x 4.6 mm, 5 µm; mobile phase: acetonitrile/water gradient; flow rate: 1 mL/min; injection: 5 µL; temperature: 50 ºC; detection: 254 nm).

### Example 3

### Synthesis of imepitoin

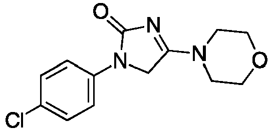

Morpholine (608.4 mL, 7.06 mol) was loaded into a mechanically stirred reactor. Ammonium chloride (125.8 g, 2.35 mol) was added to the reactor under stirring. The mixture was heated to 120 °C for 1 h. 1-(4-Chlorophenyl)imidazolidine-2,4-dione (123.8 g, 0.59 mol) was added to the solution and the mixture was heated to 130 °C. Morpholine was immediately distilled, dried by passing through a column filled with activated 3Å molecular sieves (60 g) and returned to the reactor. The mixture was maintained at 130 °C for 8 h while the morpholine was continuously distilled, dried and returned to the reactor. The reaction mixture was then cooled to 110 °C. At that temperature, toluene (620 mL) and morpholine (202 mL) were slowly added. After the addition was finished, the system was cooled to room temperature and a white solid started to precipitate as soon as the temperature decreased. When room temperature was reached, the solid was isolated by filtration. The solid was resuspended in toluene (620 mL), stirred for 1 h at room temperature, filtered and washed with water (630 mL) to obtain a white solid. The wet solid was transferred to an oven and dried under vacuum at 50 °C. The desired compound was obtained as a white solid (148.01 g, 90% yield). MS (ES+) *m*/*z*: 280 (M+H)⁺. The purity of the product was 99.8%. Purity was determined by HPLC (Luna C18 column, 250 x 4.6 mm, 5 µm; mobile phase: acetonitrile/water gradient; flow rate: 1 mL/min; injection: 5 µL; temperature: 40 ºC, detection: 254 nm)

### Example 4

The experimental procedure for the synthesis of imepitoin without drying the morpholine with 3Å molecular sieves is described herein. Morpholine (442.20 mL, 5.12 mol) was loaded into a mechanically stirred reactor. Ammonium chloride (91.43 g, 1.70 mol) was added to the reactor under stirring. The mixture was heated to 120 °C for 1 h. 1-(4-Chlorophenyl)imidazolidine-2,4-dione (90 g, 0.42 mol) was added to the solution and the mixture was heated to 130 °C. The mixture was maintained at 130 ºC for 24 hours. Morpholine (147 mL) was then added, and the mixture was cooled to 110 ºC. At that temperature, toluene (450 mL) was slowly added, and the mixture was cooled to room temperature. When room temperature was reached, the solid was isolated by filtration. The solid was then suspended in toluene (450 mL), filtered, and washed with water (450 mL). This process was repeated two more times, and the final solid was dried under vacuum at 50 ºC. The desired compound was obtained as a white solid (73.45 g, 61% yield). MS (ES+) m/z: 280 (M+H)+. The purity of the product was 99.8%. Purity was determined by HPLC (Luna C18 column, 250 x 4.6 mm, 5 µm; mobile phase: acetonitrile/water gradient; flow rate: 1 mL/min; injection: 5 µL; temperature: 40 ºC, detection: 254 nm).

It is shown that when the distillation/drying procedure of morpholine is not included in the process, 30% yield is lost, the reaction time is increased by three times and the purification process has to be repeated three times to obtain imepitoin of the same purity.

## Claims

1. A process for preparing high purity imepitoin which comprises the following steps:
a) obtention of a compound of formula (I) by an alkylation reaction 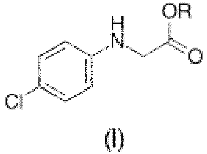 wherein R is selected from H, (C1-C4)-alkyl, allyl or benzyl, said alkylation reaction comprising the following steps:
a1) reaction of 4-chloroaniline with a base selected from potassium carbonate, sodium carbonate, potassium acetate or sodium acetate and an additive selected from potassium or sodium iodide, in a solvent selected from acetonitrile, tetrahydrofuran, dimethylformamide, acetone, or an alcohol, at room temperature, for 15-60 min.,
a2) addition of a compound of formula (II) 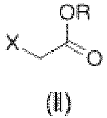 wherein X is a halogen or a leaving group, R is as previously defined, and heating at a reaction temperature between 50-80 °C, for 3-24 h,
a3) addition of water, cooling at room temperature to precipitate compound (I),
a4) distillation of the mixture ethanol/water and addition of water,
a5) isolation of compound (I) by filtration and washing with a solvent,
b) obtention of a compound of formula (III) 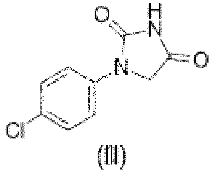 by an Urech-type hydantoin synthesis which comprises the following steps:
b1) reaction of compound of formula (I) with potassium or sodium cyanate, in a solvent selected from acetic acid or hydrochloric acid, at a reaction temperature between 100-120 °C, for 4-24 h.,
b2) cooling at room temperature, to precipitate compound (III),
b3) isolation of compound (III) by filtration, washing with a solvent, and drying,
c) obtention of imepitoin by an acid-catalyzed reversible reaction which comprises the following steps:
c1) reaction of morpholine as reactant and solvent, with ammonium chloride, at a reaction temperature between 110-140 °C, for 0.5-1.5 h,
c2) addition of the compound (III) to the mixture of morpholine and ammonium chloride at a temperature between 110-140 °C,
c3) heating the mixture to a temperature between 110-140 °C,
c4) distillation of morpholine, drying with a desiccant agent, and return of the dried morpholine to the reaction mixture that it is maintained at a temperature between 110-140 °C, for 6-12 h,
c5) cooling the reaction mixture to a temperature between 100-120 °C,
c6) slow addition of toluene and morpholine to the reaction mixture at a temperature between 100-120 °C,
c7) cooling the reaction mixture at room temperature to precipitate imepitoin,
c8) filtration of the solid and resuspension in a solvent such as toluene, ethyl acetate, acetone, acetonitrile, THF, ethanol and iso-propanol, and most preferably toluene, with stirring at 20-55 °C, preferably room temperature, for 1-2 h, preferably 1 h,
c9) filtration of the solid, washing with a solvent, preferably water, and drying under vacuum at a temperature between 40-60 °C, preferably 50

2. The process according to previous claim wherein in step a1) the base is potassium carbonate, the additive is potassium iodide, the solvent is ethanol, at room temperature, for 30 min.

3. The process according to any one of previous claims wherein in step a2) X is chlorine, R is ethyl, the T is 60 ºC and the time is 5h.

4. The process according to any one of previous claims wherein the cooling in step a3) and/or in step b2) and/or in step c8) is until room temperature.

5. The process according to any one of previous claims wherein in step a5) the washing is carried out with water.

6. The process according to any one of previous claims wherein in step b1) the compound of formula (I) is reacted with potassium cyanate, the solvent is acetic acid, at a T of 110 ºC, for 6h.

7. The process according to any one of previous claims wherein in step b3) the washing is carried out with water and the drying is carried out under vacuum at the temperature of 50

8. The process according to any one of previous claims wherein the step c1) is carried out at a T of 120 ºC for 1h.

9. The process according to any one of previous claims wherein the step c2) is carried out at a T of 120 ºC.

10. The process according to any one of previous claims wherein the heating in step c3) is carried out at a T of 130 ºC.

11. The process according to any one of previous claims wherein in step c4) the drying is carried out through a column filled with activated 3Å molecular sieves and the reaction mixture is maintained at a T of 130 ºC for 8h.
